# EUROPÄISCHE PATENTSCHRIFT

(11) **EP 1 979 038 B1**
(45) Veröffentlichungstag und Bekanntmachung des Hinweises auf die Patenterteilung: **18.05.2011**
(21) Anmeldenummer: 07703105.2
(22) Anmeldetag: 29.01.2007
(51) Int. Cl.: A61M 39/24

(54) **EINWEGE-VENTIL, INSBESONDERE NIEDERDRUCKRÜCKSCHLAGVENTIL, ZUR VERWENDUNG IN DER MEDIZINTECHNIK**
ONE-WAY VALVE, ESPECIALLY LOW-PRESSURE NON-RETURN VALVE, FOR USE IN MEDICAL TECHNOLOGY
CLAPET UNIDIRECTIONNEL, EN PARTICULIER CLAPET ANTIRETOUR BASSE PRESSION, DESTINÉ À ÊTRE UTILISÉ DANS LE DOMAINE DE LA TECHNIQUE MÉDICALE

(30) Priorität: 30.01.2006 DE 202006001474 U
(43) Veröffentlichungstag der Anmeldung: 15.10.2008
(73) Patentinhaber: Filtertek B.V., County of Limerick (IE)
(72) Erfinder: MCMAHON, Sean, Castletroy (IE)
(74) Vertreter: Beck, Alexander
(86) Internationale Anmeldenummer: PCT/EP2007/000749
(87) Internationale Veröffentlichungsnummer: WO 2007/085488

(56) Entgegenhaltungen:
- EP-A- 0 934 757
- WO-A-95/23002
- WO-A-98/39589
- US-A- 5 992 462

## Beschreibung

Die Erfindung betrifft ein Einwege-Ventil, insbesondere Niederdruckrückschlagventil, zur Verwendung in der Medizintechnik, mit einem aus einer Einlasshälfte und einer Auslasshälfte bestehenden Gehäuse, welches einen Einlasskanal und einen Auslasskanal aufweist, und mit einer in einem Druckraum angeordneten Membran, welche an einem ringförmigen Ventilsitz unter Vorspannung anliegt, der in den Auslasskanal mündet, wobei der Druckraum aus zwei Druckkammern besteht.

Einwege-Ventile dieser Art werden zur Erzeugung eines Leerlauf-Stopps, insbesondere bei Infusionsbestecken, eingesetzt, wobei die Ventile dazu verwendet werden, um zu verhindern, dass beim Leerlaufen eines mit Infusionsflüssigkeit gefüllten Vorratsbehälters Luft in die Infusionsflüssigkeit eintritt, indem ein sofortiger Leerlauf-Stopp eintritt, damit gegen Ende des Zustroms über die mit dem Venensystem eines Patienten verbundenen Leitungen keine Luft in das Venensystem gelangen kann.

Aus der DE 2919343 A1 ist beispielsweise ein Rückschlagventil zur Verwendung in einem Infusionsbesteck bekannt, welches mit einer Tropfkammer und einem Schwimmerventil ausgestattet ist, das beim Leerlaufen der Kammer das Eintreten von Luft in den Infusionsschlauch verhindert. Ausgangsseitig ist in der zur Infusionsnadel führenden flexiblen Schlauchleitung eine Rollenklemme angeordnet. Um zu verhindern, dass sich das gesamte Leitungssystem des Infusionsbestecks mit Luft füllt, wird hier ein Doppelsitzschwimmerventil mit einer Schwimmerkugel verwendet, wobei jedoch derartige Schwimmerkugelventile nicht genau genug ansprechen, so dass ein Luftzutritt in den Infusionsschlauch nicht in jedem Falle verhindert werden kann.

Aus der DE 3632412 A1 ist ferner eine weitere Leerlaufsicherung bekannt, bei der in Strömungsrichtung vor der Tropfkammer ein bei Leerlauf ansprechendes Ventil angeordnet ist. Bei diesem Ventil liegt eine Schwimmerkugel unter Reibung beim Abdichten an einer Kanalwandung an und kann bei entsprechendem Bewegungsspiel eine Luftzufuhr nicht mit absoluter Sicherheit verhindern. Die beiden vorgenannten Ventile sind nicht geeignet, die Luftzufuhr in das menschliche Venensystem sicher auszuschließen.

Aus der DE 19749562 A1 ist ferner ein Infusionsbesteck bekannt, welches aus einem hoch aufgehängten Behälter für die Infusionsflüssigkeit und einem mittels eines rohrförmigen Dorns an einen Drosselbehälter anschließbaren Tropfes sowie einer darunter auf einer flexiblen Zuleitung einstellbaren Rollenklemme und einer am Leitungsende befindlichen Injektionsnadel besteht. In Strömungsrichtung vor der Tropfkammer oder an deren Eingangsöffnung ist ein Leerlauf-Stopp vorgeschaltet, um das Eindringen von Luft in die Infusionsflüssigkeit, insbesondere gegen Ende des Zustroms der Infusionsflüssigkeit, zu verhindern. Mittels des Leerlauf-Stopps bzw. des darin eingebauten Ventils kann abhängig von einem anstehenden statischen Druck die Strömung der Infusionsflüssigkeit abgeschaltet werden. Es hat sich im praktischen Einsatz jedoch gezeigt, dass ein Leerlauf-Stopp, welcher in Abhängigkeit des statisch anstehenden Drucks der Infusionsflüssigkeit anspricht, nicht ausreicht, um sicher das Eindringen von Luft zu verhindern. Insbesondere, wenn die Möglichkeit besteht, dass die Anschlüsse durch Unachtsamkeit vertauscht werden und somit die Funktion des Leerlauf-Stoppventils nicht mehr gewährleistet ist, entsteht eine besonders gefährliche Situation, wenn dadurch Luft in das Infusionssystem eindringen kann, die beim Patienten zu einer Embolie führen kann, falls die Luft bis in das Venensystem gelangt. Es ist daher unverzichtbar, dass keine Luft mit der Infusionsflüssigkeit mitgeführt werden kann. Ein besonderer Nachteil des Ventils dieser Bauart besteht noch darin, dass im Falle des Anstehens eines statischen Überdrucks auf der Ausgangsseite keine Möglichkeit besteht, das Eindringen von Luft in das System zu verhindern.

Ein vergleichsweise kompliziert aufgebautes Sicherheitsventil für Infusionen ist noch aus der WO 97/03712 A2 bekannt, durch welches verhindert werden soll, dass Blut aus der Vene eines Patienten herausströmt, falls die Infusionsleitungen unbeabsichtigt getrennt werden.

Aus der DE 19643360 C1 ist weiterhin ein Membranventil in Dreiwege-Bauart bekannt, welches zwischen einem Ventilgehäuse und einer Membranscheibe einerseits und einem Deckel und der Membranscheibe andererseits voneinander getrennte Differenzdruckkammern aufweist, von denen die ventilgehäuseseitige Differenzdruckkammer einen ersten Anschluss für die Zuleitung, der Deckel einen zweiten Anschluss für die Zuleitung und die Differenzdruckkammern einen Anschluss für die Ableitung aufweisen.

Ein Einwege-Ventil der eingangs definierten Art ist nach einem früheren Vorschlag der Anmelderin noch aus der DE 10219994 bekannt, bei welchem die beiden Druckkammern nebeneinanderliegend angeordnet sind und mittels eines Verbindungskanals miteinander verbunden sind. Die Membran erstreckt sich hierbei entweder als zwei getrennte Teile oder einstükkig durch beide Druckkammern, wobei die Membran in der ersten Druckkammer gegen einen ersten Ventilsitz, welcher in einer Vorkammer angeordnet ist, unter Spannung anliegt und eine zentrale Öffnung aufweist, die durch den ersten Ventilsitz geschlossen wird. Ein zweiter entgegengesetzt gerichteter ringförmiger Ventilsitz, welcher mit dem Auslasskanal verbunden ist, ist in der zweiten Druckkammer angeordnet, wobei auf der dem Ventilsitz gegenüberliegenden Seite der Membran eine zweite Vorkammer vorgesehen ist, die mit der Atmosphäre in Verbindung steht. Mittels dieses bekannten Ventils wird das Eindringen von Luft in das System des Infusionsbestecks sicher sowohl bei dem Anstehen eines statischen Unterdrucks als auch eines Überdrucks in dem Auslasskanal verhindert. Ein statischer Unterdruck in dem Einlasskanal kann beispielsweise dann entstehen, wenn ein Vorratsbehälter der Infusionsflüssigkeit leergelaufen ist, wenn eine Infusionspumpe fehlerhaft arbeitet oder Luft in das System eingetreten ist. In diesem Fall schließt der Rückschlagventilteil in dem ersten Druckraum sicher. Ein Überdruck in dem Auslasskanal kann beispielsweise bei einem vorübergehenden Verschluss der Armvenen des Patienten auftreten, wenn ausgangsseitig zusätzlich eine Medikamentenpumpe angeschlossen ist. Ein Überdruck würde hier dazu führen, dass das Medikament in das mit Schwerkraft arbeitende Infusionsbesteck gepumpt wird. Dieses bekannte Einwege-Ventil ist von der Herstellung vergleichsweise aufwendig und durch die nebeneinanderliegenden Druckkammern vergleichsweise sperrig.

Der Erfindung liegt daher die Aufgabe zugrunde, unter Beibehaltung der durch das bekannte Ventil erzielten Vorteile eine raumsparende und weniger aufwendig herzustellende Konstruktion eines derartigen Ventils vorzuschlagen.

Bei einem Einwege-Ventil der eingangs genannten Art wird diese Aufgabe im Wesentlichen dadurch gelöst, dass in Strömungsrichtung vor dem durch den Ventilsitz und die Membran gebildeten Schließmechanismus ein zweiter Ventilmechanismus vorgesehen ist, welcher bei Überdruck im Eingangskanal gleichsinnig mit dem ersten Ventilmechanismus öffnet und bei Überdruck im Auslasskanal schließt.

Eine besonders vorteilhafte Ausführungsform nach der Erfindung wird hierbei dadurch geschaffen, dass der zweite Ventilmechanismus durch eine am radial äußeren Rand der Membran ausgebildete ringförmige, schüsselartige Schürze gebildet ist, welche in Durchlassrichtung schräg nach oben gerichtet ist und mit ihrer freien Außenkante an der Innenwandung des Druckraums unter Spannung anliegt und welche die Druckkammern voneinander trennt.

Es ist offensichtlich, dass dadurch das Mittel ein und derselben Membran beide Funktionen innerhalb eines Infusionsbestecks gewährleistet sind, so dass dadurch, dass gleichzeitig die Innenwandung des Gehäuses als Ventilsitz genutzt wird, eine sehr kompakte und konzentrische Bauweise erreicht werden kann, welche wesentlich weniger Raum einnimmt. Gleichzeitig ist sichergestellt, dass in den beiden oben genannten Fällen ein weiterer Transport der Infusionsflüssigkeit zuverlässig beendet wird und somit ein Eindringen von Luft enthaltender Infusionsflüssigkeit in die Venen verhindert wird. Gleichzeitig wird durch das Einwege-Ventil sichergestellt, dass bei auf der Ausgangsseite vorhandenem Überdruck, verursacht durch eine Infusions- oder Medikamentenpumpe, das Ventil schließt, indem der durch die am radial äußeren Rand der Membran ausgebildete ringförmige, schüsselartige Schürze, die dann an der Innenwandung des Gehäuses anliegt, gebildete zweite Ventilmechanismus schließt und somit ein Medikament nicht in das Infusionsbesteck gelangen kann, obwohl in diesem Falle der zum Auslasskanal führende Schließmechanismus öffnen würde. Eine, möglicherweise kurzzeitige Überdosierung wird daher sicher verhindert.

Eine vorteilhafte Weiterbildung der Erfindung kann dadurch erreicht werden, dass die Membran auf der der schüsselartigen Schürze gegenüberliegenden Seite eine nach unten weisende Ringwand aufweist, welche eine ringförmige Stützwand des Gehäuses übergreift. Dies stellt eine besonders einfache Form der Lagerung und Befestigung der Membran dar, nachdem deren Rand in Form der schüsselförmigen Schürze als Schließglied genutzt wird und somit für die Befestigung durch Einspannung der Membran nicht mehr zur Verfügung steht.

Im Einzelnen ist es von Vorteil, dass der durch die Stützwand umgebene Raum durch eine dem Ventilsitz gegenüberliegende Gehäusewandung geschlossen ist, wodurch eine dem Ventilsitz gegenüberliegende Vorkammer gebildet wird und dass die Vorkammer durch eine Gehäuseöffnung mit atmosphärem Druck beaufschlagt ist. Hierdurch wird erreicht, dass die Bauweise des Ventils extrem kompakt wird, indem der durch die Lagerung der Membran auf der Stützwand gebildete Raum als Vorkammer genutzt wird, wobei die Beaufschlagung mit Atmosphärendruck ein schnelles Ansprechen der Membran bezüglich des zum Auslasskanal führenden Ventilsitzes gewährleistet.

Im Einzelnen ist es ferner von Vorteil, dass der Einlasskanal mit einem ersten Ringraum verbunden ist, welcher durch die Stützwand bzw. die darüber geschobene Ringwand und die schüsselförmige Schürze in der Auslasshälfte begrenzt ist und die erste Druckkammer bildet. Hierdurch wird wegen der konzentrischen Bauweise weiterhin der Platzbedarf verringert.

Eine besonders vorteilhafte Ausführungsform nach der Erfindung kann dadurch geschaffen werden, dass ein zweiter Ringraum den Ventilsitz umgibt, welcher durch die schüsselartige Schürze von dem ersten Ringraum getrennt ist und die zweite Druckkammer bildet.

Im Einzelnen ist es bevorzugt, die Stützwand einstückig mit einem zwischen dem Einlassgehäuse und dem Auslassgehäuse angeordneten Bauteil auszubilden. Diese Merkmale dienen dazu, die Kosten der für die Herstellung der üblicherweise durch Spritzgießen hergestellten Ventilteile zu senken, da sich die Teile hierdurch mit einfacheren Formen herstellen lassen.

Bevorzugt ist es hierbei, dass der Bauteil die radiale Außenwand der Vorkammer bildet und außerhalb der Vorkammer Verbindungskanäle aufweist, welche den Einlasskanal mit der ersten Druckkammer verbinden. Auf diese Weise kann in einfacher Weise die dem Ventilsitz gegenüberliegende Vorkammer hergestellt werden.

Um ein sicheres Abdichten und schnelles Ansprechen zu gewährleisten, besteht ein vorteilhaftes Merkmal noch darin, dass der Querschnitt der Schürze radial nach außen abnehmend ausgebildet ist und somit in einer scharfen, an der Innenwand des Gehäuses anliegenden Kante endet.

Im Folgenden wird die Erfindung an Hand einer in den Zeichnungen beispielhaft veranschaulichten Ausführungsform näher erläutert. Es zeigt:
FIGUR 1 eine schematische Schnittansicht des Einwege-Ventils nach der Erfindung im Zustand der normalen Strömung bei einer Infusion;
FIGUR 2 eine Figur 1 entsprechende Schnittdarstellung in einem Zustand, in welchem ein Rückstrom durch das Ventil bei Anstehen eines Überdrucks im Auslass verhindert wird und
FIGUR 3 eine den Figuren 1 und 2 entsprechende Schnittansicht eines Zustands, bei welchem die Strömung verhindert wird, wenn im Ausgang ein Unterdruck vorliegt.

Die in den Zeichnungen in den verschiedenen möglichen Betriebszuständen gezeigte Ausführungsform des erfindungsgemäßen Einwege-Ventils 1, bei dem es sich um ein Niederdruckrückschlagventil zur Verwendung in der Medizintechnik, insbesondere bei Infusionen, handelt, weist eine aus Kunststoff bestehende und durch Spritzgießen hergestellte Einlasshälfte 2 und eine auf gleichem Wege hergestellte Auslasshälfte 4 auf, die zusammen ein Gehäuse 6 bilden. Die Einlasshälfte 2 enthält einen Einlasskanal 8, welcher bei dem beabsichtigten Einlassgebiet an einen, die Infusionsflüssigkeit enthaltenden Behälter (nicht dargestellt) oder eine ebenfalls nicht dargestellte Infusionspumpe angeschlossen ist. Die Auslasshälfte 4 enthält einen Auslasskanal 10, welcher über einen nicht dargestellten Schlauch mit einer Rollenklemme zu einer Infusionsnadel führt. In einem, in dem Gehäuse 6 ausgebildeten Druckraum 12 ist eine allgemein mit 14 bezeichnete Membran aus einem elastischen Werkstoff, wie Silikon oder einem thermoplastischen Elastomer, angeordnet. Die Membran 14 liegt unter Vorspannung an einem in den Druckraum vorstehenden Ventilsitz 16 an, welcher mit dem Auslasskanal 10 diesen umgebend verbunden ist.

Der Druckraum 12 besteht aus zwei durch die Membran 14 voneinander getrennten Druckkammern 18 und 20.

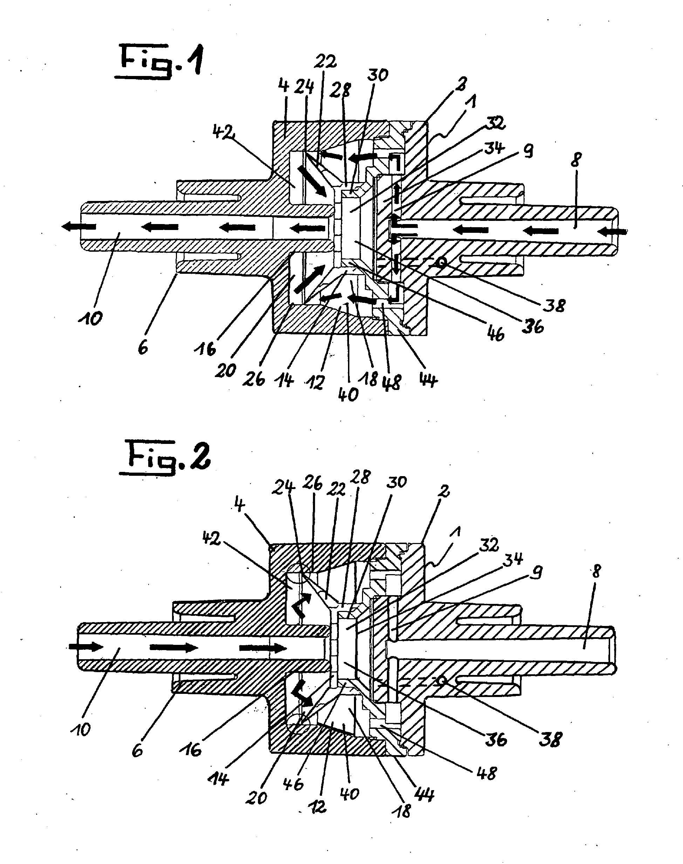

Erfindungsgemäß ist in der im Normalzustand vorliegenden Strömungsrichtung, wie sie durch die Pfeile in Figur 1 dargestellt ist, vor der durch den Ventilsitz 16 und die Membran 14 gebildeten Schließmechanismus ein zweiter Ventilmechanismus vorgesehen, welcher bei Überdruck im Eingangskanal 8 im gleichen Sinne zusammen mit dem ersten Ventilmechanismus 14, 16 öffnet und bei Überdruck im Auslasskanal 10 schließt, wenn ein derartiger Überdruck die Membran 14 von dem Ventilsitz 16 abheben sollte.

Bei der in den Zeichnungen veranschaulichten bevorzugten Ausführungsform wird der zweite Ventilmechanismus durch eine ringförmige, schüsselartige Schürze 22 gebildet, welche in der in Figur 1 dargestellten Durchlassrichtung schräg nach oben gerichtet ist. Die schüsselartige Schürze 22 ist einstückig mit der Membran 14 ausgebildet, wobei sich die Schürze 22 im Querschnitt radial nach außen verjüngt und in einer ringförmigen scharfen Außenkante 24 endet. Die Außenkante 24 liegt an der Innenwandung 26 des Druckraumes unter Vorspannung aufgrund der Elastizität der Schürze 22 an, so dass die Innenwandung 26 quasi den Ventilsitz des zweiten Ventilmechanismus bildet, der durch die schüsselartige Schürze 22 geöffnet und geschlossen werden kann. Die Schürze 22 mit der Innenwandung 26 trennt daher die Druckkammern 18 und 20 voneinander:

Auf der der schüsselartigen Schürze 22 gegenüberliegenden Seite weist die Membran 14 eine einstückige, nach unten weisende Ringwand 28 auf, welche über eine ringförmige Stützwand 30 am Gehäuse 6 übergreift. Durch diese Art der Lagerung ist eine einfache Montage möglich, indem die Membran 14 mit ihrer Ringwand 28 über die ringförmige Stützwand 30 gestülpt wird, welche allgemein dem Einlassgehäuse 2 zuzurechnen ist. Gleichzeitig wird erreicht, dass sowohl der Ventilsitz 16 als auch die Innenwandung 26, die den Ventilsitz für die Außenkante 24 der schüsselförmigen Schürze 22 bildet, tief innerhalb des Auslassgehäuses 4 angeordnet sind, so dass diese für die Funktionsfähigkeit des Ventils maßgeblichen Teile sowohl bei der Herstellung, insbesondere beim Auswerfen aus der Spritzgießform, als auch bei der Montage gegen Beschädigungen geschützt sind.

Wie gezeigt, ist der durch die Stützwand 30 umgebene Raum 32 durch eine Gehäusewandung 34 verschlossen, welche dem Ventilsitz 16 gegenüberliegt. Der durch die Stützwand 30 und die Gehäusewandung 34 geschlossene Raum bildet eine Vorkammer 36, die dem Ventilsitz 16 gegenüberliegt. Die Vorkammer 36 ist durch eine in den Zeichnungen in gestrichelten Linien dargestellte Gehäuseöffnung 38 mit der Außenluft verbunden, so dass die Vorkammer 36 mit atmosphärem Druck beaufschlagt ist. Hierdurch wird erreicht, dass sich beim Öffnen der ersten Schließeinrichtung, d.h. beim Abheben der Membran 14 vom Ventilsitz 16, kein Gegendruck aufbauen kann, so dass die Membran 14 in Minimalzeiten und leicht öffnen und schließen kann.

Der Einlasskanal 8 ist über Radialkanäle 9, die innerhalb des Einlassgehäuses 2 ausgebildet sind, mit einem ersten Ringraum 40 verbunden, welcher durch die Stützwand 30 bzw. die darüber geschobene Ringwand 28 der Membran 14 und die schüsselförmige Schürze 22 begrenzt ist und innerhalb des Auslassgehäuses 4 liegt, so dass die äußere Begrenzung des ersten Ringraums 40 durch die wandung des Auslassgehäuses 4 gebildet ist. Der erste Ringraum 40 bildet die erste Druckkammer 18.

Ein zweiter Ringraum 42 umgibt den Ventilsitz 16 und ist durch die schüsselförmige Schürze 22 von dem ersten Ringraum 40 getrennt und bildet die zweite Druckkammer 20.

Wie gezeigt, ist es aus fertigungstechnischen Gründen bevorzugt, die Stützwand 30 einstückig mit einem zwischen dem Einlassgehäuse 2 und dem Auslassgehäuse 4 angeordneten Bauteil 44 auszubilden, wobei der Bauteil 44 die radiale Außenwand 46 der Vorkammer 36 bildet und außerhalb der Vorkammer 36 Verbindungskanäle 48 aufweist, welche die Radialkanäle 9, die zum Einlasskanal 8 führen, mit der ersten Druckkammer 18 verbinden.

Der Bauteil 44 übergreift die Gehäusewandung 34 und ist mit den Wandungen des Einlassgehäuses 2 und des Auslassgehäuses 10, beispielsweise durch Ultraschallschweißen, verbunden.

Im Folgenden wird nun auf die unterschiedlichen Funktionsweisen der in den Zeichnungen veranschaulichten Ausführungsform des erfindungsgemäßen Einwege-Ventils eingegangen, wobei die jeweiligen Strömungsverhältnisse in den verschiedenen Zeichnungsfiguren durch schwarze Pfeile gekennzeichnet sind.
Figur 1 zeigt hierbei den Normalzustand der Strömung bei der Infusion, in welchem die Infusionsflüssigkeit in den Eingangskanal 8 eintritt und nach Durchströmen des Ventilabschnitts aus dem Ausgangskanal 10 ausströmt, nachdem der Öffnungsdruck des Ventils durch entsprechenden Druckanstieg im Einlasskanal 8 erreicht wurde. Hierbei strömt die Infusionsflüssigkeit vom Einlasskanal 8 über die Radialkanäle 9 und die Verbindungskanäle 48 in die erste Druckkammer 18, welche durch den Ringraum 40 gebildet ist, hebt die schüsselförmige Schürze 22 mit ihrer Außenkante 24 von der Innenwandung 26 des Druckraums 12 ab und umströmt die Außenkante 24 und erreicht die zweite Druckkammer 20. Hier wird der Mittelbereich der Membran 14 von dem Ventilsitz 16 abgehoben, so dass die Strömung in dem Auslasskanal 10 fortgesetzt wird.
Figur 2 zeigt den Zustand, in welchem im Einlasskanal kein Druck vorliegt und in welchem durch einen noch anstehenden Druck im Auslasskanal eine Rückströmung möglich wäre. Die Infusionsflüssigkeit versucht in diesem Zustand durch den Auslasskanal 10 zurückzuströmen und hebt hierbei den Mittelbereich der Membran 14 vom Ventilsitz 16 ab. In diesem Zustand werden jedoch in der zweiten Druckkammer 20 die Außenkante 24 der schüsselförmigen Schürze 22 gegen die Innenwandung 26 gedrückt, so dass eine Rückströmung verhindert wird.

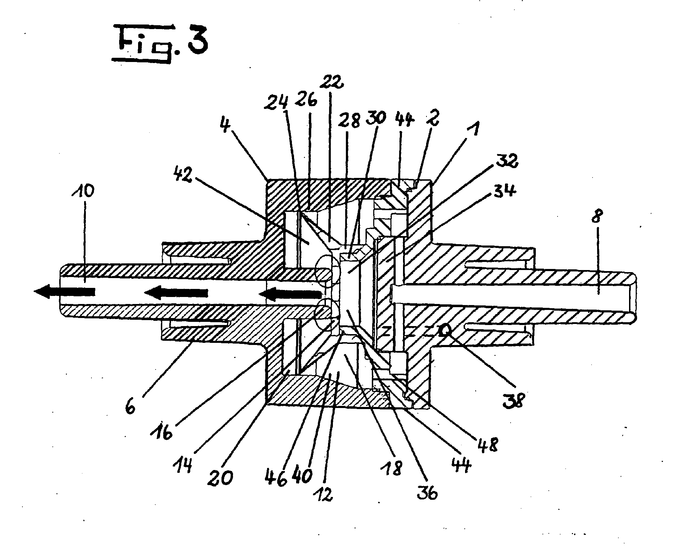

Bei dem in Figur 3 veranschaulichten Zustand ist die Funktionsweise des erfindungsgemäßen Einwege-Ventils gezeigt, wenn die Strömung verhindert werden soll, falls Unterdruck in dem Auslasskanal vorliegt. In diesem Falle wird der Mittelbereich der Membran 14 fest gegen den Ventilsitz 16 gedrückt, so dass auch hier keine unbeabsichtigte Strömung möglich ist. Es ist offensichtlich, dass sämtliche möglichen Betriebszustände in vorteilhafter Weise und sicher berücksichtigt sind.

Ein Vorteil der beschriebenen Konstruktion besteht noch darin, dass Eingangskanal 8 und Auslasskanal 10 axial fluchtend zueinander angeordnet sind, so dass eine ausgesprochen kompakte Bauweise erzielt wird. Weiterhin ist vorteilhaft, dass sämtliche Dichtflächen gegen Beschädigung während des Herstellens, des Transports und des Zusammenbaus geschützt sind. Das erfindungsgemäße Einwege-Ventil schaltet sich nicht ab, falls am Eingang ein zu hoher Druck vorliegen sollte.

Obwohl in den Zeichnungen das erfindungsgemäße Einwege-Ventil lediglich mit Schlauchanschlüssen dargestellt ist, ist es offensichtlich, dass die Anschlüsse leicht geändert werden können, um beispielsweise Luer-Lock-Anschlüsse oder Ähnliches auf beiden Seiten des Ventils vorzusehen.

Sämtliche aus der Beschreibung, den Ansprüchen und den Zeichnungen hervorgehenden Merkmale und Vorteile der Erfindung, einschließlich konstruktiver Einzelheiten und räumlicher Anordnungen, können sowohl für sich als auch in beliebiger Kombination erfindungswesentlich sein.

## Patentansprüche

1. Einwege-Ventil (1), insbesondere Niederdruckrückschlagventil, zur Verwendung in der Medizintechnik, mit einem aus einer Einlasshälfte (2) und einer Auslasshälfte (4) bestehenden Gehäuse (6), welches einen Einlasskanal (8) und einen Auslasskanal (10) aufweist und mit einer in einem Druckraum (12) angeordneten Membran (14), welche an einem ringförmigen Ventilsitz (16) unter Vorspannung anliegt, der in den Auslasskanal (10) mündet, wobei der Druckraum (12) aus zwei Druckkammern (18, 20) besteht, **dadurch gekennzeichnet, dass** in Strömungsrichtung vor dem durch den Ventilsitz (16) und die Membran (14) gebildeten Schließmechanismus (14, 16) ein zweiter Ventilmechanismus (22, 26) vorgesehen ist, welcher bei Überdruck im Eingangskanal (8) gleichsinnig mit dem ersten Schließmechanismus (14, 16) öffnet und bei Überdruck im Auslasskanal (10) schließt.

2. Einwege-Ventil nach Anspruch 1, **dadurch gekennzeichnet, dass** der zweite Ventilmechanismus (22, 26) durch eine am radial äußeren Rand der Membran (14) ausgebildete ringförmig, schüsselartige Schürze (22) gebildet ist, welche in Durchlassrichtung schräg nach oben gerichtet ist und mit ihrer freien Außenkante (24) an der Innenwandung (26) des Druckraums (12) unter Spannung anliegt und welche die Druckkammern (18, 20) voneinander trennt.

3. Einwege-Ventil nach Anspruch 1 oder 2, **dadurch gekennzeichnet, dass** die Membran (14) auf der der schüsselartigen Schürze (22) gegenüberliegende Seite eine nach unten weisende Ringwand (28) aufweist, welche eine ringförmige Stützwand (30) des Gehäuses (6) übergreift.

4. Einwege-Ventil nach Anspruch 3, **dadurch gekennzeichnet, dass** der durch die Stützwand (30) umgebene Raum (32) durch eine dem Ventilsitz (16) gegenüberliegende Gehäusewandung (34) geschlossen ist, wodurch eine dem Ventilsitz (16) gegenüberliegende Vorkammer (36) gebildet wird, und dass die Vorkammer (36) durch eine Gehäuseöffnung (38) mit atmosphärem Druck beaufschlagt ist.

5. Einwege-Ventil nach Anspruch 3 oder 4, **dadurch gekennzeichnet, dass** der Einlasskanal (8) mit einem ersten Ringraum (40) verbunden ist, welcher durch die Stützwand (30) bzw. die darüber geschobene Ringwand (28) und die schüsselförmige Schürze (22) in der Auslasshälfte (4) begrenzt ist und die erste Druckkammer (18) bildet.

6. Einwege-Ventil nach Anspruch 5, **dadurch gekennzeichnet, dass** ein zweiter Ringraum (42) den Ventilsitz (16) umgibt, welcher durch die schüsselförmige Schürze (22) von dem ersten Ringraum (40) getrennt ist und die zweite Druckkammer (20) bildet.

7. Einwege-Ventil nach einem der vorstehenden Ansprüche, **dadurch gekennzeichnet, dass** die Stützwand (30) einstückig mit einem zwischen dem Einlassgehäuse (2) und dem Auslassgehäuse (4) angeordneten Bauteil (44) ausgebildet ist.

8. Einwege-Ventil nach Anspruch 7, **dadurch gekennzeichnet, dass** der Bauteil (44) die radiale Außenwand (46) der Vorkammer (36) bildet und außerhalb der Vorkammer (36) Verbindungskanäle (48) aufweist, welche den Einlasskanal (8) mit der ersten Druckkammer (18) verbinden.

9. Einwege-Ventil nach einem der vorstehenden Ansprüche, **dadurch gekennzeichnet, dass** der Querschnitt der Schürze (22) radial nach außen abnehmend ausgebildet ist.

## Claims

1. Check valve (1), especially low pressure check valve, for the use in the medical technique, having a housing consisting of an inlet half (2) and an outlet half (4), which is provided with an inlet channel (8) and an outlet channel (10) and having a diaphragm (14) provided in a pressure space (12) which is contacting an annular valve seat (16) under pretension, the valve seat (16) opening into the outlet channel (10), wherein the pressure space (12) is consisting of two pressure chambers (18, 20), **characterised in that** a second valve mechanism (22, 26) is provided in the direction of flow ahead of the closing mechanism (14, 16) formed by the valve seat (16) and the diaphragm (14) which at an over-pressure in the entry channel (8) is opening in the same sense with the first closing mechanism (14, 16) and which at an over-pressure in the outlet channel (10) is closing.

2. Check valve according to claim 1, **characterised in that** the second valve mechanism (22, 26) is formed by an annular cup-like skirt (22) formed at the radially outer margin of the diaphragm (14) which in the opening direction is angularly directed upwardly and which with its free outer edge (24) is contacting the interior wall (26) of the pressure space (12) under pretension and which is separating the pressure chambers (18, 20) from each other.

3. Check valve according to claim 1 or 2, **characterised in that** the diaphragm (14) on the side opposite to the cup-like skirt (24) is having an annular wall (28) directed downwardly which is overlapping an annular supporting wall (30) of the housing (6).

4. Check valve according to claim 3, **characterised in that** the space (32) enclosed by the supporting wall (30) is closed by a housing wall (34) opposite to the valve seat (16), whereby a pre-chamber (36) opposite to the valve seat (16) is formed and that the pre-chamber (36) is connected to atmospheric pressure by an opening (38) in the housing.

5. Check valve according to claim 3 or 4, **characterised in that** the inlet channel (8) is connected with a first annular space (40) which is limited by the supporting wall (30) or the overlapping annular wall (28), respectively, and the cup-shaped skirt (22) in the exit half (4) and is forming the first pressure chamber (18).

6. Check valve according to claim 5, **characterised in that** a second annular space (42) is surrounding the valve seat (16) which is separated from the first annular space (40) by the cup-shaped skirt (22) and which is forming the second pressure chamber (20).

7. Check valve according to any of the preceding claims, **characterised in that** the supporting wall (30) is monolithic with a member (44) positioned between the inlet half (2) and the outlet half (4).

8. Check valve according to claim 7, **characterised in that** the member (44) is forming the radial outer wall (46) of the pre-chamber (36) and is having connection channels (48) beyond the pre-chamber (36) which are connecting the inlet channel (8) with the first pressure chamber (18).

9. Check valve according to any of the preceding claims, **characterised in that** the cross-section of the skirt (22) is radially decreasing to its outer margin.

## Revendications

1. Clapet anti-retour (1), en particulier clapet anti-retour basse pression, pour l'utilisation dans le domaine de la technique médicale, ayant un boîtier constitué d'une moitié d'entrée (2) et d'une moitié de sortie (4), qui est pourvue d'un conduit d'entrée (8) et d'un conduit de sortie (10) et ayant un diaphragme (14) prévu dans un espace de pression (12) avec lequel vient en contact un siège de soupape annulaire (16) précontraint, le siège de soupape (16) ouvrant dans le conduit de sortie (10), l'espace de pression (12) consistant en deux chambres de pression (18, 20), **caractérisé en ce qu'**un deuxième mécanisme de soupape (22, 26) est prévu dans la direction d'écoulement en avant du mécanisme de fermeture (14, 16) formé par le siège de soupape (16) et le diaphragme (14), qui, sous l'effet d'une surpression dans le conduit d'entrée (8), s'ouvre dans le même sens que le premier mécanisme de fermeture (14, 16) et qui se ferme dans le cas d'une surpression dans le conduit de sortie (10).

2. Clapet anti-retour selon la revendication 1, **caractérisé en ce que** le deuxième mécanisme de soupape (22, 26) est formé par une jupe de type coupelle annulaire (22) formée au niveau de la marge radialement extérieure du diaphragme (14) qui, dans la direction d'ouverture, est orientée angulairement vers le haut et qui, avec son bord extérieur libre (24), vient en contact avec la paroi intérieure (26) de l'espace de pression (12) précontraint et qui sépare les chambres de pression (18, 20) l'une de l'autre.

3. Clapet anti-retour selon la revendication 1 ou 2, **caractérisé en ce que** le diaphragme (14) sur le côté opposé de la jupe de type coupelle (24) a une paroi annulaire (28) orientée vers le bas, qui chevauche une paroi de support annulaire (30) du boîtier (6).

4. Clapet anti-retour selon la revendication 3, **caractérisé en ce que** l'espace (32) renfermé par la paroi de support (30) est fermé par une paroi de boîtier (34) opposée au siège de soupape (16), une préchambre (36) opposée au siège de soupape (16) étant formée et **en ce que** la préchambre (36) est connectée à la pression atmosphérique par une ouverture (38) dans le boîtier.

5. Clapet anti-retour selon la revendication 3 ou 4, **caractérisé en ce que** le conduit d'entrée (8) est connecté à un premier espace annulaire (40) qui est limité par la paroi de support (30) ou par la paroi annulaire de chevauchement (28), respectivement, et la jupe en forme de coupelle (22) dans la moitié de sortie (4), et qui forme la première chambre de pression (18).

6. Clapet anti-retour selon la revendication 5, **caractérisé en ce qu'**un deuxième espace annulaire (42) entoure le siège de soupape (16), lequel est séparé du premier espace annulaire (40) par la jupe en forme de coupelle (22) et qui forme la deuxième chambre de pression (20).

7. Clapet anti-retour selon l'une quelconque des revendications précédentes, **caractérisé en ce que** la paroi de support (30) est monolithique, avec un organe (44) positionné entre la moitié d'entrée (2) et la moitié de sortie (4).

8. Clapet anti-retour selon la revendication 7, **caractérisé en ce que** l'organe (44) forme la paroi radiale extérieure (46) de la préchambre (36) et présente des conduits de connexion (48) au-delà de la préchambre (36), lesquels relient le conduit d'entrée (8) à la première chambre de pression (18).

9. Clapet anti-retour selon l'une quelconque des revendications précédentes, **caractérisé en ce que** la section transversale de la jupe (22) diminue radialement jusqu'à sa marge extérieure.
